# FASCICULE DE BREVET EUROPEEN

(11) **EP 0 538 100 B1**
(45) Date de publication et mention de la délivrance du brevet: **06.02.2002**
(21) Numéro de dépôt: 92402742.8
(22) Date de dépôt: 08.10.1992
(51) Int. Cl.: C07D 215/54, C07D 471/04, C07C 205/44

(54) **Procédé de préparation de dérivés de l'acide fluoro quinoléine carboxylique-3**
Verfahren zur Herstellung von Fluoro-Chinolin-3-Carbonsäurederivate
Process for the preparation of fluoro-quinolin-3-carboxylic acid derivatives

(30) Priorité: 10.10.1991 FR 9112480
(43) Date de publication de la demande: 21.04.1993
(73) Titulaire: Aventis Pharma S.A., 92160 Antony (FR)
(72) Inventeur: Daubie, Christophe, F-75005 Paris (FR); Legrand, Jean-Jacques, F-75013 Paris (FR); Pemberton, Clive, Romfold, Essex RM3 0XB (GB)

(56) Documents cités:
- EP-A- 0 236 140
- EP-A- 0 379 412
- FR-A- 2 225 166

## Description

La présente invention concerne la préparation de dérivé de l'acide fluoro quinoléine carboxylique-3 de formule générale : 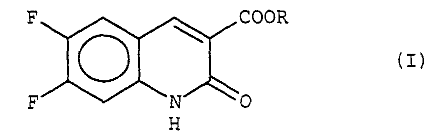 dans laquelle R est un atome d'hydrogène ou un radical alcoyle ainsi que ses sels lorsqu'ils existent.

Dans le brevet US 4 970 213 ont été décrites des acides fluoro-6 quinoléine carboxyliques de structure : 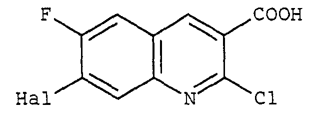 dans laquelle Hal est un atome de fluor ou de chlore, utiles comme intermédiaires pour la préparation de benzonaphtyridines-1,8 ayant une activité antimicrobienne.

Dans la demande de brevet français 2 225 166 a été décrite la préparation de pyrimidines condensées, par l'intermédiaire d'amino-2 quinoléine-3-carboxamide (ou de l'ester ou l'acide correspondant) ou encore selon une autre alternative, par l'intermédiaire de chloro-2 quinoléine-3 carboxylate d'alcoyle.

Dans la demande européene 236 140 ont été décrits des dérivés de carbostyrile substitués par un groupe azido ou un groupe azoté et par des radicaux alcoyle, alcoyloxy, hydroxy, halogène ...

Les dérivés de la quinoléine préparés selon la présente invention sont également utiles pour la préparation de dérivés de la benzonaphtyridine-1,8 antimicrobiens, mais permettent d'obtenir des rendements améliorés et évitent ainsi de mettre en oeuvre le procédé en passant intermédiairement par des produits instables.

Dans la formule générale (I) lorsque R représente un radical alcoyle, ce dernier est droit ou ramifié et contient 1 à 4 atomes de carbone.

Selon la présente invention les dérivés de la quinoléine de formule générale (I) peuvent être préparés par cyclisation en milieu réducteur acide d'un dérivé nitré de formule générale : 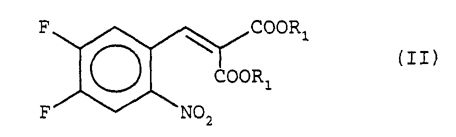 dans laquelle les radicaux R₁ sont des radicaux alcoyle tels que définis précédemment pour R, suivie éventuellement de la libération de la fonction acide si l'on veut obtenir un dérivé de la quinoléine pour lequel R est un atome d'hydrogène.

Le traitement en milieu acide s'effectue en présence de fer, à une température comprise entre 0 et 130°C, au moyen de tout acide organique ou minéral qui n'altère pas le reste de la molécule. A titre d'exemple on opère au moyen de l'acide acétique ou de l'acide formique, il est également possible d'opérer au moyen d'acide chlorhydrique dilué ou d'acide sulfurique dilué en milieu hydro-alcoolique. Il est bien entendu que le choix de l'acide est fonction du produit attendu. Dans le cas où l'on veut obtenir l'acide de formule générale (I), il est avantageux d'opérer dans un acide plus fort, dans des conditions où l'hydrolyse de l'ester s'effectue simultanément. Il est bien entendu que dans les cas où l'on a obtenu l'ester et où l'on veut obtenir l'acide de formule générale (I) pour lequel R est un atome d'hydrogène, l'hydrolyse de l'ester peut être également mise en oeuvre après la réaction de cyclisation, par toute méthode connue pour obtenir un acide à partir d'un ester sans toucher au reste de la molécule.

Le cas échéant l'hydrolyse de l'ester s'effectue en milieu acide, par exemple en présence d'acide chlorhydrique, d'acide sulfurique ou méthanesulfonique. Elle peut aussi être effectuée en milieu hydroalcoolique basique (soude, potasse par exemple).

Le dérivé nitré de formule générale (II) peut être préparé par action d'un dérivé de l'acide malonique de formule générale :

R₁OCO-CH₂-COOR₁ (III)

dans laquelle R₁ et R₂ sont définis comme précédemment, sur un dérivé du nitrobenzaldéhyde de formule :

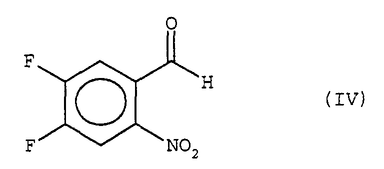

La réaction s'effectue généralement en milieu basique [par exemple en présence d'un bicarbonate alcalin (bicarbonate de sodium), d'un hydrure (hydrure de sodium) ou d'un alcoolate à une température comprise entre 0 et 150°C, dans un solvant organique tel qu'un anhydride (anhydride acétique par exemple) ou tel qu'un amide (diméthyl-formamide, N-méthylpyrrolidone par exemple) en opérant en présence de tamis moléculaires ou de tout autre agent desséchant ou encore dans un mélange de solvants comme un mélange solvant aprotique polaire/anhydride acétique (diméthylformamide/anhydride acétique, N-méthyl pyrrolidone/anhydride acétique par exemple). Il est également possible d'opérer en milieu biphasique. Il n'est pas indispensable d'isoler le produit de formule générale (II) pour le mettre en oeuvre dans la réaction suivante.

Le fluoronitrobenzaldéhyde de formule générale (IV) est obtenu par nitration du fluorobenzaldéhyde de formule :

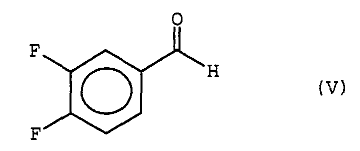

La réaction s'effectue avantageusement par l'acide nitrique concentré sous forme d'un mélange sulfonitrique, ou d'un mélange acide nitrique/acide acétique, à une température comprise entre 0 et 90°C.

Selon l'invention les dérivés de la fluoro quinoléine de formule générale (I) sont utiles comme intermédiaires de synthèse pour la préparation de dérivés de la benzo[b]naphtyridine-1,8 de formule générale : 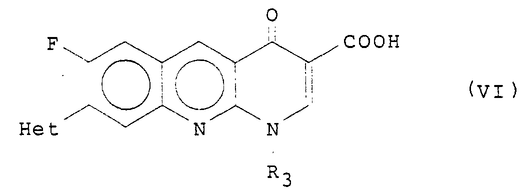 dans laquelle soit R₃ (qui représente un radical alcoyle, fluoroalcoyle, cycloalcoyle contenant 3 à 6 atomes de carbone, alcoyloxy ou alcoylamino) et Het (qui est un radical hétérocyclyle azoté), sont tels que définis pour les substituants en position -1 et -8, dans la demande européenne EP 431 991 et le brevet US 5 004 745, soit R₃ est un atome d'hydrogène ou un radical alcoyle, fluoroalcoyle, carboxyalcoyle, cycloalcoyle contenant 3 à 6 atomes de carbone, fluorophényle, difluorophényle, alcoyloxy ou alcoylamino,et Het est un radical azétidinyle-1 substitué [en position -3 par un radical R₄ qui peut être un atome d'hydrogène ou un radical hydroxy, amino, alcoylamino dont la partie alcoyle est éventuellement substituée par un radical amino ou hydroxy ou peut représenter un radical dialcoylamino dont les parties alcoyle peuvent éventuellement former avec l'atome d'azote auquel elles sont rattachées, un hétérocycle à 5 ou 6 chaînons contenant éventuellement un autre hétéroatome choisi parmi l'azote, l'oxygène ou le soufre, ou peut représenter un radical cycloalcoylamino contenant 3 à 6 chaînons, ou un radical alcanoylamino, N-alcoyl N-alcanoyl amino ou aminoalcoyl phénylamino, et substitué en positions -2 et -3 par des radicaux R₅ et R₆ identiques ou différents qui re présentent des atomes d'hydrogène, des radicaux alcoyle, alcènyle contenant 2 à 4 atomes de carbone, phényle, phényle substitué par un atome d'halogène, ou par un radical alcoyle, alcoyloxy, hydroxy, nitro, amino, alcoylamino, dialcoylamino ou halogénoalcoyle, ou bien substitué en position -2 par des radicaux R₅ et R₆ qui représentent des radicaux alcoyle], étant entendu que les radicaux alcoyle et alcanoyle cités ci-dessus sont droits ou ramifiés et contiennent 1 à 4 atomes de carbone.

Ces dérivés de la benzonaphtyridine sont utiles comme antimicrobiens.

Les dérivés de la quinoléine selon la présente invention sont également utiles pour la préparation des intermédiaires de formule générale (XI) définis ci-après qui sont les précurseurs de dérivés de la benzonaphtyridine de formule générale (VI).

Selon l'invention les benzo[b]naphtyridines-1,8 de formule générale (VI) peuvent être obtenues à partir des produits selon l'invention en opérant de la manière suivante :

On prépare un chloro fluoro ester de formule générale : 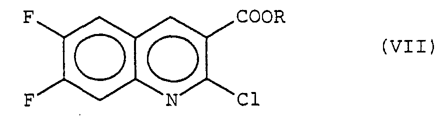 dans laquelle R est défini comme précédemment, par chloration du dérivé de l'acide fluoroquinoléine carboxylique de formule générale (I) dont le cas échéant la fonction acide est préalablement protégée (lorsque R est une atome d'hydrogène).

La chloration s'effectue au moyen des agents de chloration connus qui n'altèrent pas le reste de la molécule. Notamment on opère par action du chlorure de phosphoryle, du chlorure de sulfuryle ou du pentachlorure de phosphore à une température comprise entre 0 et 150°C.

Lorsque l'on veut obtenir l'acide de formule générale (VII) dans laquelle R est un atome d'hydrogène, on effectue l'hydrolyse de l'ester obtenu par toute méthode connue qui n'altère pas le reste de la molécule. La protection et l'élimination du radical protecteur peuvent être réalisées par tout groupement compatible et dont la mise en oeuvre et l'élimination n'altèrent pas le reste de la molécule. Notamment, on opère selon les méthodes décrites par T.W. GREENE, Protective Groups in Organic Synthesis, A. Wiley Interscience Publication (1981), ou par Mc OMIE, Protective Groups in Organic Chemistry, Plenum Press (1973).

Le dérivé de la benzonaphtyridine de formule générale (VI) peut être obtenu à partir de l'acide chloro fluoro quinoléine carboxylique de formule générale (VII) dans laquelle R est un atome d'hydrogène, selon ou par analogie avec la méthode décrite dans la demande européenne EP 431 991 et le brevet US 5 004 745 ou US 4 970 213.

Le dérivé de la benzonaphtyridine de formule générale (VI) peut également être obtenu à partir de l'ester de formule générale (VII) en opérant comme suit :

Une amine de formule générale :

R₃-NH-CH₂-CH₂-R₇ (VIII)

dans laquelle R₃ est défini comme précédemment et R₇ est un radical alcoyloxycarbonyle, cyano, carbamoyle, alcoylcarbamoyle, benzylcarbamoyle, hydroxyéthylcarbamoyle, dialcoylaminoéthylcarbamoyle ou dialcoylcarbamoyle dont les parties alcoyle peuvent éventuellement former avec l'atome d'azote auquel elles sont rattachées, un hétérocycle à 5 ou 6 chaînons contenant éventuellement un autre hétéroatome choisi parmi l'oxygène, le soufre ou l'azote et éventuellement substitué sur l'azote par un radical alcoyle, (les radicaux alcoyle étant droits ou ramifiés et contenant 1 à 4 atomes de carbone), est condensée sur la chloro fluoro quinoléine de formule générale (VII) dans laquelle R est un radical alcoyle, de manière à obtenir un fluoro ester de formule générale : 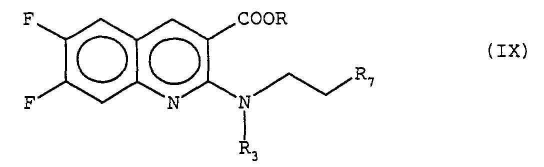 dans laquelle R, R₃ et R₇ sont définis comme précédemment.

La condensation s'effectue en milieu basique dans un solvant organique tel qu'un hydrocarbure aromatique (toluène par exemple), un amide (diméthylformamide, N-méthyl pyrrolidone par exemple), un éther (tétrahydrofuranne par exemple), un sulfoxyde (diméthylsulfoxyde par exemple), un solvant chloré (dichlorométhane, dichloroéthane, chlorobenzène par exemple) ou un alcool à une température comprise entre -10 et 120°C.

A titre d'exemple les bases utilisées peuvent être choisies parmi les carbonates alcalins (carbonate de sodium ou de potassium), les alcoolates ou un hydrure alcalin (hydrure de sodium).

Il est entendu que, dans l'alternative où le symbole R₃ représente un radical carboxyalcoyle, ce dernier est protégé préalablement à la réaction. L'élimination du radical protecteur est effectuée de préférence après la réaction d'oxydation, sur le dérivé de la benzonaphtyridine de formule générale (XI) décrit ci-après. La protection et la libération de la fonction acide s'effectuent selon les méthodes habituelles qui n'altèrent pas le reste de la molécule. Notamment selon les méthodes qui ont été citées précédemment.

La fluoro quinoléine de formule générale (IX) est cyclisée en milieu basique pour préparer la tétrahydro-1,2,3,4 benzo[b]naphtyridine-1,8 de formule générale : 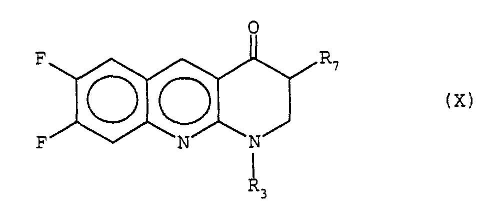 dans laquelle R₃ et R₇ sont définis comme précédemment.

La réaction s'effectue à une température comprise entre -70 et 120°C en présence d'une base comme un alcoolate (éthylate de sodium, méthylate de sodium, t.butylate de potassium par exemple), un hydrure alcalin (hydrure de sodium par exemple), ou encore un hydroxyde alcalin en opérant par transfert de phase. On opère avantageusement dans un solvant aprotique polaire (par exemple diméthylformamide, tétrahydrofuranne) ou dans un alcool (éthanol, méthanol par exemple) dans un glyme ou dans un glycol (éthylène glycol par exemple). Lorsque l'on effectue la réaction par transfert de phase, on opère avantageusement dans un solvant chloré comme le chlorure de méthylène, la base étant en solution dans la phase aqueuse.

La tétrahydro-1,2,3,4 benzo[b]naphtyridine-1,8 de formule générale (X) est oxydée pour préparer la benzo[b]naphtyridine-1,8 de formule générale : 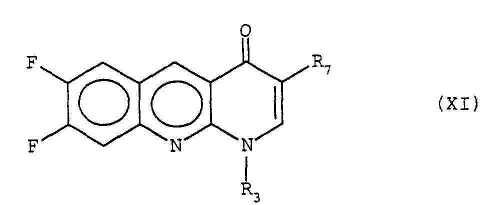 dans laquelle R₃ et R₇ sont définis comme précédemment.

L'oxydation s'effectue par l'eau oxygénée, éventuellement en présence d'iodure de potassium, dans un solvant organique tel qu'un alcool (éthanol par exemple), à une température comprise entre 0 et 120°C. Il est également possible d'opérer en milieu biphasique dans un mélange eau/solvant chloré (dichlorométhane, dichloroéthane...).

L'hétérocycle Het est condensé sur la benzo[b]naphtyridine-1,8 de formule générale (XI) ou l'acide correspondant pour préparer un dérivé de la benzonaphtyridine de formule générale (VI), en opérant selon ou par analogie avec les méthodes décrites dans la demande européenne EP 431 991 et le brevet US 5 004 745 puis le cas échéant par transformation de l'ester, de l'amide ou du nitrile obtenu en un acide de formule générale (VI). Les dérivés de la benzonaphtyridine de formule générale (VI) sont des antimicrobiens dont les activités ont été décrites dans la demande européenne et le brevet américain cités ci-dessus. Les dérivés de la benzonaphtyridine de formule générale (VI) pour lesquels Het est un radical azétidinyle présentent également des propriétés antibactériennes. Ils manifestent une activité remarquable in vitro et in vivo sur les germes gram positifs et aussi sur les germes gram négatifs. In vitro, ils sont actifs à une concentration comprise entre 0,06 et 4 µg/cm³ sur staphylococcus aureus IP 8203 et à une concentration comprise entre 0,25 et 20 µg/cm³ sur Escherichia coli souche NIHJ JC2. In vivo, ils sont actifs sur les infections expérimentales de la souris à staphylococcus aureus IP 8203 à des doses comprises entre 10 et 200 mg/kg par voie orale.

Les produits préparés selon la présente invention, ainsi que les produits auquels ils conduisent, peuvent être éventuellement purifiés par des méthodes physiques telles que la cristallisation ou la chromatographie.

Les intermédiaires de formule générale (I) préparés selon la présente invention pour lesquels R est un atome d'hydrogène peuvent être transformés en sels métalliques ou en sels d'addition avec les bases azotées selon les méthodes connues en soi. Ces sels peuvent être obtenus par action d'une base métallique (par exemple alcaline ou alcalino-terreuse), de l'ammoniac ou d'une amine sur un produit selon l'invention dans un solvant approprié tel qu'un alcool, un éther ou l'eau ou par réaction d'échange avec un sel d'un acide organique. Le sel formé précipite après concentration éventuelle de sa solution, il est séparé par filtration, décantation ou lyophilisation.

Comme exemples de sels, peuvent être cités les sels avec les métaux alcalins (sodium, potassium, lithium) ou avec les métaux alcalino-terreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl phénéthylamine, NN'-dibenzyléthylènediamine, diphénylènediamine, benzhydrylamine, quinine, choline, arginine, lysine, leucine, dibenzylamine), ainsi que les sels d'addition avec des acides minéraux (chlorhydrates, bromhydrates, sulfates, nitrates, phosphates) ou organiques (succinates, fumarates, maléates, méthanesulfonates, p.toluène-sulfonates, iséthionates).

Les exemples suivants illustrent la présente invention.

### EXEMPLE

A une solution de 56,5 g de difluoro-3,4 nitro-6 benzaldéhyde dans 92 cm³ d'anhydride acétique, on ajoute, sous agitation, en dix minutes, 62,8 g de malonate d'éthyle et 51 g de bicarbonate de sodium. La suspension est maintenue 1 heure à environ 20°C puis chauffée 3 heures à une température d'environ 75°C. A cette température on verse, en 30 minutes, 400 cm³ d'acide acétique glacial puis 65 cm³ d'eau. On laisse la température se stabiliser à 50°C environ et l'on agite encore 30 minutes à cette température. On ajoute 39 g de fer en poudre, par fractions, en 2 heures, au mélange réactionnel. La température s'élève à environ 85°C et la suspension est maintenue 1 heure supplémentaire à cette température. Les sels de fer formés sont essorés à 80°C environ, puis lavés par 2 fois 150 cm³ d'acide acétique glacial. Le filtrat et les phases acides de lavage sont réunis et additionnés à 700 cm3 d'eau. le précipité obtenu est essoré à 20°C environ et lavé par 3 fois 500 cm³ d'eau. Le produit humide obtenu est séché sous pression réduite (20 kPa) à 50°C environ. On obtient 53,5 g d'éthoxycarbonyl-3 difluoro-6,7 carbostyrile sous forme d'un solide crème fondant à 242°C.

Le difluoro-3,4 nitro-6 benzaldéhyde est préparé de la manière suivante

A 520 cm3 d'acide sulfurique, sous agitation, refroidi, à 0°C, on ajoute, en 30 minutes, 60 cm³ d'acide nitrique fumant. A la solution obtenue, on ajoute, en 30 minutes, à environ 0°C, 100 g de difluoro-3,4 benzaldéhyde. On laisse remonter la température à environ 20°C et agite encore 3 heures à cette température. Après refroidissement à environ 5°C, le mélange réactionnel est versé, en 30 minutes, sous forte agitation, dans 1200 g de glace pilée. On laisse la température remonter à environ 20°C et extrait par 2 fois 600 cm³ de toluène. Les extraits organiques réunis sont lavés par 3 fois 1 000 cm³ d'eau, et concentrées sous pression réduite (20kPa) à 50°C. On obtient 113 g de difluoro-3,4 nitro-6 benzaldéhyde sous forme d'une huile brune qui est utilisée telle quelle dans les synthèses ultérieures. Un échantillon purifié de difluoro-3,4 nitro-6 benzaldéhyde donne les caractéristiques suivantes :
PE(6,66 Pa)=46°C.
Spectre de RMN(400 MHz, DMSO, T=298°K)
10,20 ppm(1H, 1s) ; 8,5 ppm(1H, 1q) ; 8,05 ppm(1H, 1q)

Les produits selon l'invention peuvent être utilisés de la manière suivante :

### Exemple d'utilisation 1

A 200 cm³ de chlorure de phosphoryle, on ajoute en 10 minutes, sous agitation, à 20°C, 50 g d'éthoxycarbonyl-3 difluoro-6,7 carbostyrile. La suspension est chauffée à une température voisine de 70°C et maintenue à cette température pendant 3 heures. Après refroidissement à environ 10°C, la solution obtenue est versée, sous agitation, dans un mélange de 1 000 cm³ d'eau et 1 000 g de glace pilée. On laisse la température remonter à environ 20°C et extrait par 2 fois 500 cm³ de dichlorométhane. Les extraits organiques réunis sont lavés par 1 000 cm³ d'eau, 1 000 cm³ d'eau additionnés de bicarbonate de sodium jusqu'à pH=7, séchés sur sulfate de sodium, filtrés et concentrés à sec sous pression réduite (20 kPa) à environ 40°C. On obtient 45,6 g de chloro-2 éthoxycarbonyl-3 difluoro-6,7 quinoléïne sous forme d'un solide beige fondant à 108°C qui est utilisé sans autre purification pour les étapes ultérieures. La chloro-2 éthoxycarbonyl-3 difluoro-6,7 quinoléïne est convertie en acide chloro-2 difluoro-6,7 quinoléïne carboxylique-3 selon les méthodes habituelles et peut conduire ainsi aux dérivés de la benzo[b]naphtyridine-1,8 décrits dans le brevet US 4 970 213.

### Exemple d'utilisation 2

### Préparation de l'éthoxycarbonyl-3 difluoro-6,7 (N-méthyl N-b éthoxycarbonyléthyl) amino-2 quinoléïne :

A une solution de 72 g de chloro-2 éthoxycarbonyl-3 difluoro-6,7 quinoléïne préparée comme décrit à l'exemple d'utilisation 1 et 45,1 g de N-méthyl, N-b éthoxycarbonyléthyl amine dans 750 cm³ de toluène on ajoute 56,2 g de carbonate de sodium. La suspension obtenue est chauffée à environ 90°C puis agitée 4 heures à cette température. Le mélange réactionnel est ensuite refroidi à environ 20°C puis lavé par 3 fois 400 cm³ d'eau. La phase organique est concentrée à sec sous pression réduite (20kPa) à environ 50°C. On obtient 94 g d'éthoxycarbonyl-3 difluoro-6,7 (N-méthyl N-b éthoxycarbonyléthyl) amino-2 quinoléïne sous forme d'une huile utilisée sans autre purification pour les étapes ultérieures.

### Préparation de l'éthoxycarbonyl-3 difluoro-7,8 méthyl-1 oxo-4 tétrahydro-1,2,3,4 benzo[b]naphtyridine-1,8 :

A une solution de 26,6 g d'éthylate de sodium portée à reflux dans 900 cm³ d'éthanol absolu on ajoute en 80 minutes, une solution de 94 g d'éthoxycarbonyl-3 difluoro-6,7 (N-méthyl N-b éthoxycarbonyléthyl) amino-2 quinoléïne dans 300 cm³ d'éthanol absolu. La suspension obtenue, toujours à reflux, est agitée 15 minutes supplémentaires. On verse ensuite, en 30 minutes, 38 cm³ d'acide acétique glacial. Le mélange réactionnel est agité 15 minutes supplémentaires puis on verse en 45 minutes, toujours à reflux, 500 cm³ d'eau. La suspension obtenue est refroidie à 20°C environ. Le précipité est essoré à 20°C environ et lavé par 2 fois 300 cm³ d'eau. Le produit humide est séché sous pression réduite (20 kPa) à environ 60°C. On isole 71,5 g d'éthoxycarbonyl-3 difluoro-7,8 méthyl-1 oxo-4 tétrahydro-1,2,3,4 benzo[b]naphtyridine-1,8 sous forme d'un solide jaune fondant à 188°C.

### Préparation de l'éthoxycarbonyl-3 difluoro-7,8 méthyl-1 oxo-4 dihydro-1,4 benzo[b]naphtyridine-1,8 :

A une suspension de 71 g d'éthoxycarbonyl-3 difluoro-7,8 méthyl-1 oxo-4 tétrahydro-1,2,3,4 benzo[b]naphtyridine-1,8 dans 1000 cm³ d'éthanol, on ajoute sous agitation à environ 20°C une solution de 3,78 g d'iodure de potassium dans 20 cm³ d'eau. La suspension est chauffée à 77°C et on ajoute à cette température, en 60 minutes, 30 cm³ d'eau oxygénée à 33 % en poids. Le mélange réactionnel est maintenu à reflux 30 minutes supplémentaires puis est refroidi à environ 20°C. A cette température on verse, en 5 minutes, une solution de 11,4 g de thiosulfate de sodium dans 50 cm³ d'eau. Le précipité obtenu est essoré à 20°C environ et lavé par 2 fois 300 cm³ d'eau. Le produit humide obtenu est séché sous pression réduite (20 kPa) à environ 60°C. On isole 73 g d'éthoxy-3 difluoro-7,8 méthyl-1 oxo-4 dihydro-1,4 benzo[b]naphtyridine-1,8 sous forme d'un solide blanc fondant à une température supérieure à 270°C.

### Exemple d'utilisation 3

### Préparation de l'éthoxycarbonyl-3 difluoro-6,7 (N-éthyl N-b éthoxycarbonyléthyl) amino-2 quinoléïne :

A une solution de 10 g de chloro-2 éthoxycarbonyl-3 difluoro-6,7 quinoléïne préparée comme décrit à l'exemple d'utilisation 1 et 9,7 g de N-éthyl, N-b éthoxycarbonyléthyl amine dans 120 cm³ de toluène, on ajoute 7,8 g de carbonate de sodium. La suspension obtenue est chauffée à environ 90°C puis agitée 4 heures à cette température. Le mélange ractionnel est ensuite refroidi à environ 20°C puis lavé par 3 fois 100 cm³ d'eau. La phase organique est concentrée à sec sous pression réduite (20 kPa) à environ 50°C. On obtient 13 g d'éthoxycarbonyl-3 difluoro-6,7 (N-éthyl N-b éthoxycarbonyléthyl) amino-2 quinoléïne sous forme d'une huile qui est utilisée sans autre purification pour les étapes ultérieures.

### Préparation de l'éthoxycarbonyl-3 difluoro-7,8 éthyl-1 oxo-4 tétrahydro-1,2,3,4 benzo[b]naphtyridine-1,8 :

A une solution de 16,1 g d'éthylate de sodium porté à reflux dans 600 cm³ d'éthanol absolu on verse, en 60 minutes, une solution de 68 g d'éthoxycarbonyl-3 difluoro-6,7 (N-éthyl N-b éthoxycarbonyléthyl) amino-2 quinoléïne dans 200 cm³ d'éthanol absolu. La suspension obtenue, toujours, à reflux, est agitée 60 minutes supplémentaires. On verse, ensuite, en 30 minutes, 20 cm³ d'acide acétique glacial. Le mélange réactionnel est agité 15 minutes supplémentaires puis on verse en 45 minutes, toujours à reflux, 400 cm³ d'eau. La suspension obtenue est refroidie à 20°C environ. Le précipité obtenu est essoré à 20°C environ et lavé par deux fois 200 cm³ d'eau. Le produit humide est séché sous pression réduite (20 kPa) à environ 50°C. On isole 52,4 g d'éthoxycarbonyl-3 difluoro-7,8 éthyl-1 oxo-4 tétrahydro-1,2,3,4 benzo[b]naphtyridine-1,8 sous forme d'un solide jaune d'or fondant à 152°C.

### Préparation de l'éthoxycarbonyl-3 difluoro-7,8 éthyl-1 oxo-4 dihydro-1,4 benzo[b]naphtyridine-1,8 :

A une suspension de 33 g d'éthoxycarbonyl-3 difluoro-7,8 éthyl-1 oxo-4 tétrahydro-1,2,3,4 benzo[b]naphtyridine-1,8 dans 1000 cm³ d'éthanol, on ajoute sous agitation à environ 20°C une solution de 1,7 g d'iodure de potassium dans 10 cm³ d'eau. La suspension est chauffée à 77°C et on verse à cette température, en 30 minutes, 12,7 cm³ d'eau oxygénée à 33 % en poids. Le mélange réactionnel ests maintenu à reflux 30 minutes supplémentaires puis est refroidi à environ 20°C. A cette température on verse en 5 minutes une solution de 6 g de thiosulfate de sodium dans 20 cm³ d'eau. Le précipité obtenu est essoré à 20°C environ et lavé par deux fois 150 cm³ d'eau. Le produit humide obtenu est séché sous pression réduite (20 kPa) à environ 50°C. On isole 28,7 g d'éthoxy-3 difluoro-7,8 éthyl-1 oxo-4 dihydro-1,4 ben-zo[b]naphtyridine-1,8 sous forme d'une solide jaune clair fondant à 270°C.

### Exemple d'utilisation 4

### Préparation de l'éthoxycarbonyl-3 difluoro-6,7 (N-cyclopropyl N-b éthoxycarbonyléthyl) amino-2 quinoléïne :

A une solution de 3,48 g de chloro-2 éthoxycarbonyl-3 difluoro-6,7 quinoléïne préparée comme décrit à l'exemple d'utilisation 1 et 3 g de N-cyclopropyl N-b éthoxycarbonyléthyl amine dans 10 cm³ de toluène, on ajoute 3 g de carbonate de sodium. La suspension obtenue est chauffée à reflux puis agitée 15 heures à cette temprérature. Le mélange réactionnel est ensuite refroidi à environ 20°C puis on ajoute 30 cm³ d'eau et 4,5 cm³ d'acide acétique. Après décantation, le mélange réactionnel est lavé par 2 fois 10 cm³ d'eau. La phase organique est concentrée à sec sous pression réduite (20 kPa) à environ 50°C. On obtient 3,3 g d'éthoxycarbonyl-3 difluoro-6,7 (N-cyclopropyl N-b éthoxycarbonyléthyl) amino-2 quinoléïne brute sous forme d'une huile qui est utilisée sans autre purification pour l'étape ultérieure.

### Préparation de l'éthoxycarbonyl-3 difluoro-7,8 cyclopropyl-1 oxo-4 tétrahydro-1,2,3,4 benzo[b]naphtyridine-1,8 :

A une solution de 1,6 g d'éthylate de sodium porté à reflux dans 40 cm³ d'éthanol absolu on ajoute, en 60 minutes, une solution d'éthoxycarbonyl-3 difluoro-6,7 (N-cyclopropyl N-b éthoxycarbonyléthyl) amino-2 quinoléïne dans 20 cm³ d'éthanol absolu. La solution obtenue est agitée 60 minutes supplémentaires, à reflux. On verse ensuite, en 10 minutes, 2,6 cm³ d'acide acétique glacial. Le mélange réactionnel est agité 15 minutes supplémentaires puis on verse en 5 minutes, toujours à reflux, 26 cm³ d'eau. La suspension obtenue est refroidie à 20°C environ. Le précipité est essoré à 20°C environ et lavé par deux fois 10 cm³ d'eau. Le produit humide est séché sous pression réduite (20 kPa) à environ 60°C. on isole 1,25 g d'éthoxycarbonyl-3 difluoro-7,8 cyclopropyl-1 oxo-4 tétrahydro-1,2,3,4 benzo[b]naphtyridine-1,8 brute sous forme d'un solide jaune fondant à 172°C.

### Préparation de l'éthoxycarbonyl-3 difluoro-7,8 cyclopropyl-1 oxo-4 dihydro-1,4 benzo[b]naphtyridine-1,8 :

A une suspension de 1 g d'éthoxycarbonyl-3 difluoro-7,8 cyclopropyl-1 oxo-4 tétrahydro-1,2,3,4 benzo[b]naphtyridine-1,8 dans 14 cm³ d'éthanol, on ajoute sous agitation à environ 20°C une solution de 0,053 g d'iodure de potassium dans 0,5 cm³ d'eau. La suspension est chauffée à 77°C et on verse à cette température, en 5 minutes, 0,5 cm³ d'eau oxygénée à 33 % en poids. Le mélange réactionnel est maintenu à reflux 60 minutes supplémentaires puis refroidi à environ 20°C. A cette température on verse en 5 minutes, 1,06 cm³ d'une solution 1N de thiosulfate de sodium. Le précipité obtenu est essoré à 20°C environ et lavé par 2 fois 10 cm³ d'eau. Le produit humide obtenu est séché sous pression réduite (20 kPa) à environ 60°C. On isole 0,7 g d'éthoxy-3 difluoro-7,8 cyclopropyl-1 oxo-4 dihydro-1,4 benzo[b]naphtyridine-1,8 brute sous forme d'un solide blanc ocre fondant à 210°C.

### Exemple d'utilisation 5

### L'éthoxycarbonyl-3 difluoro-6,7 (N-méthyl N-b-cyanoéthyl amino)-2 quinoléine est préparée de la manière suivante :

A une solution de 16,3 g de chloro-2 éthoxycarbonyl-3 difluoro-6,7 quinoléine préparée comme décrit à l'exemple d'utilisation 1 et 10 g de N-méthyl N-b-cyanoéthyl amine dans 160 cm³ de toluène, on ajoute 19,08 g de carbonate de sodium. La suspension obtenue est chauffée à reflux puis agitée 4 heures à cette température. Le mélange réactionnel est ensuite refroidi à environ 20°C puis lavé par 3 fois 50 cm³ d'eau. La phase organique est concentrée à sec sous pression réduite (20 kPa) à environ 50°C. On obtient 19,17 g d'éthoxycarbonyl-3 difluoro-6,7 (N-méthyl N-b-cyanoéthyl amino)-2 quinoléine sous forme d'une huile qui est utilisée sans autre purification pour les étapes ultérieures.

### La cyano-3 difluoro-7,8 méthyl-1 oxo-4 tétrahydro-1,2,3,4 benzo[b]naphtyridine-1,8 est préparée de la manière suivante :

A une solution de 8,74 g de terbutylate de potassium dans 200 cm³ de tétrahydrofuranne refroidi à -10°C on verse, en 60 minutes, une solution de 19,17 g d'éthoxycarbonyl-3 difluoro-6,7 (N-méthyl N-b-cyanoéthyl amino)-2 quinoléine dans 50 cm³ de tétrahydrofuranne. La suspension obtenue est agitée toujours à -10°C durant 30 minutes supplémentaires. On verse ensuite 4 cm³ d'acide acétique glacial. Le tétrahydrofuranne est évaporé sous pression réduite (20 kPa). Le mélange brut réactionnel est repris par 200 cm³ d'un mélange hydroalcoolique éthanol/eau (70/30 vol/vol). Le précipité obtenu est filtré, lavé 2 fois par 50 cm3 d'eau, puis séché sous pression réduite (20 kPa). On isole 16,1 g de cyano-3 difluoro-7,8 méthyl-1 oxo-4 tétrahydro-1,2,3,4 benzo[b]naphtyridine-1,8 sous forme d'un solide jaune d'or fondant à 144°C.

### La cyano-3 difluoro-7,8 méthyl-1 oxo-4 dihydro-1,4 benzo[b]naphtyridine-1,8 est préparée de la manière suivante :

A une suspension de 8,6 g de cyano-3 difluoro-7,8 méthyl-1 oxo-4 tétrahydro-1,2,3,4 benzo[b]naphtyridine-1,8 dans 350 cm³ d'éthanol, on ajoute sous agitation à environ 20°C une solution de 0,47 g d'iodure de potassium dans 5 cm³ d'eau. La suspension est chauffée à 77°C et additionnée à cette température, en 10 minutes, de 4 cm³ d'eau oxygénée à 33 % en poids. Le mélange réactionnel est maintenu à reflux 30 minutes supplémentaires puis est refroidi à environ 20°C. A cette température on ajoute, en 5 minutes, 10 cm³ d'une solution de thiosulfate de sodium 1N. Le précipité obtenu est essoré à 20°C environ et lavé par deux fois 20 cm³ d'eau. Le produit humide obtenu est séché sous pression réduite (20 kPa) à environ 50°C. On isole 8 g de cyano-3 difluoro-7,8 méthyl-1 oxo-4 dihydro-1,4 benzo[b]naphtyridine-1,8 sous forme d'un solide jaune clair fondant à 380°C.

### La cyano-3 fluoro-7 méthyl-1 (méthyl-4 pipérazinyl-1)-8 oxo-4 dihydro-1,4 benzo[b]naphtyridine-1,8 est préparée de la manière suivante:

Une suspension de 2,1 g de cyano-3 difluoro-7,8 méthyl-1 oxo-4 dihydro-1,4 benzo[b]naphtyridine-1,8 dans 100 cm³ de diméthylsulfoxyde est chauffée à 80°C en présence de 2 cm³ de N-méthyl pipérazine. Le mélange réactionnel est maintenu à cette température durant 8 heures. La solution obtenue est refroidie à température ambiante et agitée à cette température durant 15 heures. Le précipité formé est filtré, lavé par 3 fois 20 cm³ d'eau, et séché sous vide (20 kPa) à 50°C. On obtient 2,6 g de cyano-3 fluoro-7 méthyl-1 (méthyl-4 pipérazinyl-1)-8 oxo-4 benzo[b]naphtyridine-1,8 sous forme d'un précipité jaune fondant à 335°C.

### L'acide fluoro-7 méthyl-1 (méthyl-4 pipérazinyl-1)-8 oxo-4 dihydro-1,4 benzo[b]naphtyridine-1,8 carboxylique-3 est préparé de la manière suivante :

Une suspension de 2 g de cyano-3 fluoro-7 méthyl-1 (méthyl-4 pipérazinyl-1)-8 oxo-4 dihydro-1,4 benzo[b]naphtyridine-1,8 est chauffé à reflux dans 40 cm³ d'acide chlorhydrique 12N. Le mélange réactionnel est maintenu à cette température durant 15 heures. La solution obtenue est refroidie à température ambiante. Le produit qui cristallise est filtré, lavé à l'eau jusqu'à neutralité, et séché sous pression réduite (20 kPa) à 50°C. On obtient 1,5 g d'acide fluoro-7 méthyl-1 (méthyl-4 pipérazinyl-1)-8 oxo-4 dihydro-1,4 benzo[b]naphtyridine-1,8 carboxylique-3 monochlorhydrate sous forme de cristaux jaunes fondant à 290°C (décomposition).

### Exemple d'utilisation 6

### L'éthoxycarbonyl-3 difluoro-6,7 [N-méthyl N-b (N',N'-diméthylaminocarbonyléthyl) amino]-2 quinoléine est préparée de la manière suivante :

A une solution de 26 g de chloro-2 éthoxycarbonyl-3 difluoro-6,7 quinoléine préparée comme décrit à l'exemple d'utilisation 1 et 25 g de N-méthyl N-b (N',N'-diméthylaminocarbonyléthyl) amine dans 300 cm³ de toluène, on ajoute 31 g de carbonate de sodium. La suspension obtenue est chauffée à reflux puis agitée 2 heures 30 minutes à cette température. Le mélange réactionnel est ensuite refroidi à environ 20°C puis lavé par 3 fois 100 cm³ d'eau. La phase organique est concentrée à sec sous pression réduite (20 kPa) à environ 50°C. On obtient 35 g d'éthoxycarbonyl-3 difluoro-6,7 [N-méthyl N-b (N',N'-diméthylaminocarbonyl éthyl) amino]-2 quinoléine sous forme d'une huile qui est utilisée sans autre purification pour les étapes ultérieures.

### La N,N-diméthyl difluoro-7,8 méthyl-1 oxo-4 tétrahydro-1,2,3,4 benzo[b]naphtyridine-1,8 carboxamide-3 est préparée de la manière suivante :

A une solution de 15,7 g de terbutylate de potassium dans 150 cm³ de tétrahydrofuranne refroidi à 0°C on ajoute, en 75 minutes, une solution de 35 g d'éthoxycarbonyl-3 difluoro-6,7 N-méthyl N-b (N',N'-diméthylaminocarbonyl éthyl) amino-2 quinoléine dans 150 cm³ de tétrahydrofuranne. La suspension obtenue est ensuite agitée à 0°C durant 30 minutes supplémentaires puis on ajoute 8 cm³ d'acide acétique glacial. Le tétrahydrofuranne est évaporé sous pression réduite (20 kPa). Le mélange brut réactionnel est repris par 200 cm³ d'un mélange hydroalcoolique éthanol/eau (70/30 vol/vol). Le précipité obtenu est filtré, lavé 3 fois par 100 cm³ d'eau, puis séché sous vide (20 kPa). On isole 25 g de N,N-diméthyl difluoro-7,8 méthyl-1 oxo-4 tétrahydro-1,2,3,4 benzo[b]naphtyridine-1,8 carboxamide-3 sous forme d'un solide jaune citron fondant à 206°C .

### La N,N-diméthyl difluoro-7,8 méthyl-1 oxo-4 dihydro-1,4 benzo[b] naphtyridine-1,8 carboxamide-3 est préparée de la manière suivante :

A une suspension de 25 g de N,N-diméthyl difluoro-7,8 méthyl-1 oxo-4 tétrahydro-1,2,3,4 benzo[b]naphtyridine-1,8 carboxamide-3 dans 1000 cm³ d'éthanol, on ajoute sous agitation à environ 20°C une solution de 1,35 g d'iodure de potassium dans 10 cm³ d'eau. La suspension est chauffée à 77°C et on verse à cette température, en 20 minutes, 25 cm³ d'eau oxygénée à 33 % en poids. Le mélange réactionnel est maintenu à reflux 1 heure 30 minutes supplémentaire puis est refroidi à environ 20°C. A cette température on coule,en 5 minutes, 30 cm³ d'une solution de thiosulfate de sodium 1N. Le précipité obtenu est essoré à 20°C environ et lavé par deux fois 60 cm³ d'eau. Le produit humide obtenu est séché sous pression réduite (20 kPa) à environ 50°C. On isole 19,5 g de N,N-diméthyl difluoro-7,8 méthyl-1 oxo-4 dihydro-1,4 benzo[b]naphtyridine-1,8 carboxamide-3 sous forme d'un solide jaune clair fondant à 324°C.

Une suspension de 2,96 g de difluoro-7,8 N,N-diméthyl oxo-4 méthyl-1 dihydro-1,4 benzo[b]naphtyridine-1,8 carboxamide-3, de 1,12 g de méthyl-1 pipérazine et de 1,55 g de carbonate de potassium dans 100 cm³ de diméthylsulfoxyde est chauffée 5 heures, sous agitation, à environ 80°C. Après refroidissement à environ 20°C, le mélange réactionnel est additionné de 100 cm³ d'eau ; l'insoluble est essoré, lavé par 2 fois 30 cm³ d'eau et 2 fois 30 cm³ d'éthanol.

On obtient 2,3 g de N,N diméthyl fluoro-7 méthyl-1 (méthyl-4 pipérazinyl-1)-8 oxo-4 dihydro-1,4 benzo[b]naphtyridine-1,8 carboxamide-3 sous forme d'un solide jaune se décomposant à 275°C.

Une solution de 0,5 g de N,N-diméthyl fluoro-7 méthyl-1 (méthyl-4 pipérazinyl-1)-8 oxo-4 dihydro-1,4 benzo[b]naphtyridine-1,8 carboxamide-3 dans 10 cm³ d'acide chlorhydrique aqueux 6N est chauffée, sous agitation à environ 95°C, pendant 5 heures. Après refroidissement à environ 20°C, l'insoluble est essoré, lavé par 3 fois 20 cm³ d'eau et 2 fois 10 cm³ d'éthanol.

Le produit obtenu est mis en suspension dans 30 cm³ d'eau ; on ajoute 0,6 cm³ de potasse aqueuse N et agite 1 heure à environ 20°C. L'insoluble est essoré, lavé par 2 fois 20 cm³ d'eau et 2 fois 10 cm³ d'éthanol. Après recristallisation dans 15 cm³ diméthyformamide, on obtient 0,15 g d'acide fluoro-7 méthyl-1 (méthyl-4 pipérazinyl-1)-8 oxo-4 dihydro-1,4 benzo[b]naphtyridine-1,8 carboxylique-3 sous forme d'un solide jaune se décomposant à 354°C.

### Exemple d'utilisation 7

### L'éthoxycarbonyl-3 difluoro-6,7 (N-méthyl N-b aminocarbonyléthyl amino)-2 quinoléine est préparée de la manière suivante :

A une solution de 4 g de chloro-2 éthoxycarbonyl-3 difluoro-6,7 quinoléine préparée comme décrit à l'exemple d'utilisation 1 et 3 g de N-méthyl N-b-aminocarbonyléthyl amine dans 40 cm³ de toluène, on ajoute 4,4 g de carbonate de sodium. La suspension obtenue est chauffée à reflux puis agitée 2 heures 30 minutes à cette température. Le mélange réactionnel est ensuite refroidi à environ 20°C puis lavé par 3 fois 25 cm³ d'eau. La phase organique est concentrée à sec sous pression réduite (20 kPa) à environ 50°C. On obtient 4,7 g d'éthoxycarbonyl-3 difluoro-6,7 (N-méthyl N-b-aminocarbonyléthyl amino)-2 quinoléine sous forme d'une huile qui est utilisée sans autre purification pour les étapes ultérieures.

### La carboxamide-3 difluoro-7,8 méthyl-1 oxo-4 tétrahydro-1,2,3,4 benzo[b]naphtyridine-1,8 est préparée de la manière suivante :

A une solution de 1,8 g de terbutylate de potassium dans 50 cm³ de tétrahydrofuranne refroidi à 0°C on ajoute, en 30 minutes, une solution de 4,23 g d'éthoxycarbonyl-3 difluoro-6,7 (N-méthyl N-b- aminocarbonyléthyl amino)-2 quinoléine dans 20 cm³ de tétrahydrofuranne. La suspension obtenue est ensuite agitée à 0°C durant 30 minutes supplémentaires puis on verse 2 cm³ d'acide acétique glacial. Le tétrahydrofuranne est évaporé sous pression réduite (20 kPa). Le brut réactionnel est repris par 10 cm³ d'un mélange hydroalcoolique éthanol/eau ( 70/30 vol/vol). Le précipité obtenu est filtré, lavé 3 fois par 10 cm³ d'eau, puis séché sous pression réduite (20 kPa). On isole 1,6 g de carboxamide-3 difluoro-7,8 méthyl-1 oxo-4 tétrahydro-1,2,3,4 benzo[b]naphtyridine-1,8 sous forme d'un solide jaune fondant à 182°C.

### La carboxamide-3 difluoro-7,8 méthyl-1 oxo-4 dihydro-1,4 benzo[b] naphtyridine-1,8 est préparée de la manière suivante :

A une suspension de 1,3 g de carboxamide-3 difluoro-7,8 méthyl-1 oxo-4 tétrahydro-1,2,3,4 benzo[b]naphtyridine-1,8 dans 25 cm³ d'éthanol, on ajoute sous agitation à environ 20°C une solution de 0,1 g d'iodure de potassium dans 1 cm³ d'eau. La suspension est chauffée à 77°C et additionnée à cette température, en 5 minutes, de 1,5 cm³ d'eau oxygénée à 33 % en poids . Le mélange réactionnel est maintenu à reflux 1 heure 30 minutes supplémentaire puis est refroidi à environ 20°C. A cette température on ajoute 1 cm³ d'une solution de thiosulfate de sodium 1N. Le précipité obtenu est essoré à 20°C environ et lavé par deux fois 5 cm³ d'eau. Le produit humide obtenu est séché sous pression réduite (20 kPa) à environ 50°C. On isole 1,1 g de carboxamide-3 difluoro-7,8 méthyl-1 oxo-4 dihydro-1,4 benzo[b]naphtyridine-1,8 sous forme d'un solide orangé fondant à 318°C .

Une suspension de 1,3 g de difluoro-7,8 méthyl-1 oxo-4 dihydro-1,4 benzo[b]naphtyridine-18 carboxamide-3, de 0,54 g de méthyl-1 pipérazine et de 0,75 g de carbonate de potassium dans 25 cm³ de diméthylsulfoxyde est chauffée à environ 80°C pendant 6 heures. Après refroidissement à une température voisine de 20°C, le mélange réactionnel est additionné de 100 cm³ d'eau. L'insoluble est essoré, lavé par 2 fois 20 cm³ d'eau et 2 fois 20 cm³ d'éthanol.

Le produit obtenu est chromatographié sur 20 g de gel de silice (0,063-0,200 mm) en suspension dans un mélange de dichlorométhane à 10 % de méthanol. On élimine de impuretés réactionnelles par élution avec 500 cm³ de ce mélange de solvants. Le produit attendu est ensuite élué par 500 cm³ du même mélange de solvants. Après concentration à sec, sous pression réduite (20 kPa) à envion 40°C, le résidu solide est recristallisé dans 25 cm³ de diméthylformamide, essoré et lavé par 2 fois 30 cm³ d'éthanol à environ 70°C.

On obtient 0,6 g de fluoro-7 méthyl-1 (méthyl-4 pipérazine-1)-8 oxo-4 dihydro-1,4 benzo[b]naphtyridine-1,8 carboxamide-3 sous forme d'un solide jaune se décomposant à 265°C.

L'acide fluoro-7 (méthyl-4 pipérazinyl-1)-8 méthyl-1 oxo-4 dihydro-1,4 benzo[b]naphtyridine-1,8 carboxylique-3 est préparé dans les conditions de l'exemple d'utilisation 2, mais à partir de 0,3 g de fluoro-7 méthyl-1 (méthyl-4 pipérazinyl-1)-8 oxo-4 dihydro-1,4 benzo[b]naphtyridine-1,8 carboxamide-3. Après refroidissement à environ 20°C, le mélange réactionnel est additionné de 50 cm³ d'eau ; l'insoluble est essoré et lavé par 2 fois 10 cm³ d'eau.

Le produit obtenu est mis en suspension dans 20 cm³ d'eau, additionné de 0,4 cm³ d'une solution de potasse aqueuse N et agité pendant 1 heure à environ 20°C. L'insoluble est essoré, lavé par 3 fois 10 cm³ d'eau, 2 fois 10 cm³ d'éthanol et recristallisé dans 20 cm³ de diméthylformamide.

On obtient 0,17 g d'acide fluoro-7 méthyl-1 (méthyl-4 pipérazinyl-1)-8 oxo-4 dihydro-1,4 benzo[b]naphtyridine-1,8 carboxylique-3, sous forme d'un solide jaune se décomposant à 354°C.

## Revendications

1. Procédé de préparation d'un dérivé de la quinoléine de formule générale: 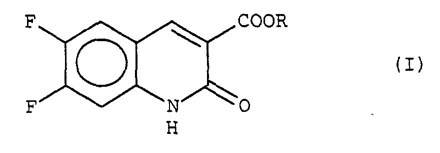 dans laquelle R est un atome d'hydrogène ou un radical alcoyle, **caractérisé en ce que** l'on effectue les étapes suivantes :
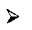 action d'un dérivé de l'acide malonique de formule générale :
R₁OCO-CH₂-COOR₁ (III)
dans laquelle les radicaux R₁ sont des radicaux alcoyle tels que définis ci-dessus pour R sur un dérivé du nitrobenzaldéhyde de formule : 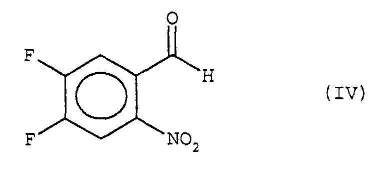
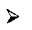 cyclisation en milieu acide du dérivé nitré de formule générale: 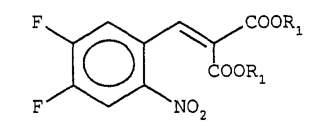 dans laquelle R₁ est défini comme ci-dessus, suivie éventuellement de la libération de la fonction acide si l'on veut obtenir un dérivé de la quinoléine pour lequel R est un atome d'hydrogène.

2. Le difluoro-3,4 nitro-6 benzaldéhyde.

## Patentansprüche

1. Verfahren zur Herstellung eines Chinolin-Derivates der allgemeinen Formel (I) 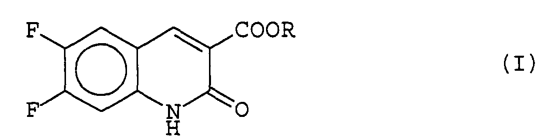 in der R ein Wasserstoffatom oder ein Rest Alkyl ist, **dadurch gekennzeichnet, daß** man die folgenden Stufen durchführt:
- Einwirkung eines Malonsäure-Derivates der allgemeinen Formel (III)
R₁OCO-CH₂-COOR₁ (III)
in der die Reste R₁ wie oben für R definierte Reste Alkyl sind, auf ein Nitrobenzaldehyd-Derivat der Formel (IV) 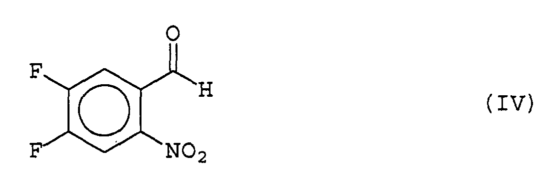
- Cyclisierung im sauren Medium des nitrierten Derivates der allgemeinen Formel 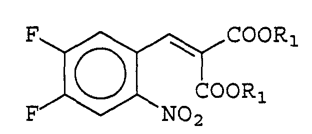 in der R₁ wie oben definiert ist, gegebenenfalls gefolgt von der Freisetzung der Säurefunktion, wenn man wünscht, ein Chinolin-Derivat zu erhalten, worin R ein Wasserstoffatom ist.

2. 3,4-Difluor-6-nitro-benzaldehyd.

## Claims

1. Process for the preparation of a quinoline derivative of general formula: 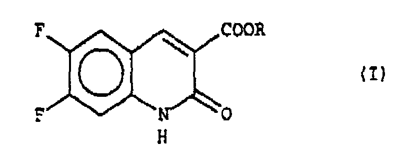 in which R is a hydrogen atom or an alkyl radical, **characterized in that** the following stages are carried out:
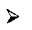 reaction of a malonic acid derivative of general formula:
R₁OCO-CH₂-COOR₁ (III)
in which the R₁ radicals are alkyl radicals as defined above for R, with a nitrobenzaldehyde derivative of formula: 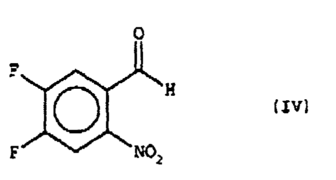
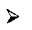 cyclization in acidic medium of the nitro derivative of general formula: 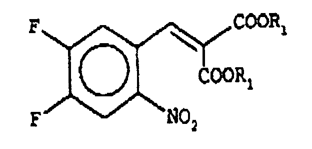 in which R₁ is defined as above, optionally followed by the release of the acidic functional group if it is desired to obtain a quinoline derivative for which R is a hydrogen atom.

2. 3,4-Difluoro-6-nitrobenzaldehyde.
